# FASCICULE DE BREVET EUROPEEN

(11) **EP 1 056 741 B1**
(45) Date de publication et mention de la délivrance du brevet: **22.01.2003**
(21) Numéro de dépôt: 99905980.1
(22) Date de dépôt: 24.02.1999
(51) Int. Cl.: C07D 319/12

(54) **PROCEDE DE PRODUCTION D'ESTERS CYCLIQUES DIMERIQUES**
VERFAHREN ZUR HERSTELLUNG VON DIMEREN ZYKLISCHEN ESTERN
METHOD FOR PRODUCING DIMERIC CYCLIC ESTERS

(30) Priorité: 24.02.1998 BE 9800142
(43) Date de publication de la demande: 06.12.2000
(73) Titulaire: Brussels Biotech, 1180 Bruxelles (BE)
(72) Inventeur: COSZACH, Philippe, B-1180 Bruxelles (BE); VAN GANSBERGHE, Frédéric, B-1180 Bruxelles (BE); BOGAERT, Jean-Christophe, B-1180 Bruxelles (BE); DECOUVREUR, Serge, B-1180 Bruxelles (BE); VAN NIEUWENHOVE, Thierry, B-1180 Bruxelles (BE); DEGEE, Philippe, B-1190 Bruxelles (BE)
(74) Mandataire: Vandeberg, Marie-Paule L.G.
(86) Numéro de dépôt international: BE9900027
(87) Numéro de publication internationale: WO99043669

(56) Documents cités:
- WO-A-93/19058
- WO-A-95/09879
- DE-A- 4 407 035
- US-A- 5 326 887

## Description

### Domaine de l'invention

La présente invention concerne un procédé de production d'esters cycliques dimériques, en particulier de lactide ou de glycolide. Ces esters cycliques sont des composés de formule générale : 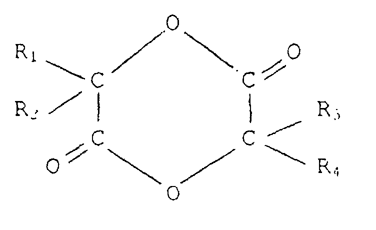 où R₁, R₂, R₃ et R₄ peuvent être soit un hydrogène, soit un groupement aliphatique substitué ou non, soit un groupement aromatique ayant de 1 à 10 atomes de carbone Si R₁ et R₃ sont un hydrogène et R₂ et R₄ un méthyle, on obtient le lactide. Si R₁ à R₄ représentent un hydrogène on obtient le glycolide.

Ces esters cycliques forment une classe très utile de composés pouvant être transformé en matière polymérique. De tels matériaux polymériques sont particulièrement utiles pour la préparation de substances plastiques biodégradables dans l'environnement et se résorbant lorsqu'ils sont utilisés dans des applications médicales. Les polymères fabriqués au départ de la polymérisation d'esters cycliques, tel que le lactide par exemple, sont particulièrement appréciés car ils peuvent être dégradés au cours du temps par hydrolyse aqueuse sous la plupart des conditions environnementales. Les unités d'hydroxyacides (par ex acide lactique) en résultant ou, des oligomères de ce dernier, sont ensuite facilement absorbés par les micro-organismes dans l'environnement et, ainsi convertis sous aérobiose en dioxyde de carbone et eau et, sous anaérobiose, en dioxyde de carbone et méthane. Les esters cycliques sont également particulièrement utiles en tant que plastifiant et intermédiaires pour la fabrication d'agents actifs de surface.

L'acide lactique ou acide hydroxy-2-propanoique est un acide carboxylique α-hydroxylé qui peut être produit par fermentation de divers substrats carbonés purs (glucose, saccharose, lactose, ...) ou impurs (produits d'hydrolyse de l'amidon, mélasses, lactosérum,...) à l'aide de micro-organismes tels que les bactéries des genres *Lactobacillus, Pediococcus, Lactococcus* et *Streptococcus,* ou certains champignons tels que les *Rhizopus Oryzae*. D'autres voies d'obtention de l'acide lactique sont connues de l'homme de l'art, via des transformations chimiques de réactifs issus de la pétrochimie telles que l'hydrolyse du lactonitrile lui-même obtenu au départ d'acétaldéhyde, la chloration et l'hydrolyse d'acide propionique ou encore via la nitration du propène.

L'acide lactique en solution diluée dans l'eau doit ensuite être purifié soit par précipitation sous forme de lactates métalliques suivie d'une réaction de neutralisation à l'acide sulfurique (Maesato K., Komori A., Taki Chem. Co, JP 6,272,646 (25/09/85)), soit par estérification avec un alcool, distillation et hydrolyse de l'ester formé (Boroda T.A., Polovko V.N., Chistyakova E.A., Pishch.Prom. 1966, 4, 35-8), soit par électrodialyse ( Jacquement J.C., Rhone-Poulenc, DE 1,957,395 (14/11/68)). Un procédé de purification plus récent consiste à extraire l'acide lactique par extraction liquide-liquide au moyen d'au moins un solvant organique non miscible à l'eau, en présence ou non d'au moins une base de Lewis telle qu'une amine tertiaire. Selon ce procédé, l'acide lactique doit être récupéré dans une seconde étape par une extraction liquide-liquide en retour. Cette étape permet de retransférer l'acide lactique dans l'eau (Baniel A.M., Blumberg R., Hadju K.,IMI, DE 2,329,480 (19/06/72); Baniel A.M., Miles Lab., EP 49,429 (06/10/80)). Enfin, l'acide lactique sous forme acide et/ou sous forme de lactate d'ammonium ou encore de lactate métallique peut être purifié par passage sur des colonnes échangeuses d'ions cationiques et/ou anioniques (Napierala W., Siminski M., Przem Ferment. Rolny. 1972, 16(12), 4-10, Shkurino O.V., Dauksha V.E., Khim-Farm.Zh. 1986, 20(10), 1375-77 ; Maesato K., Komori A., Taki Chem.Co., JP 6,272,646 (25/09/85) ; Obara H.,Shimadzu corp., JP 63,188,632 (30/01/87); Obara H., Shimadzu Corp, JP 0,191,788 (30/09/87) ; Zeleneva N.A., Ivanova E.V., Karpushina I.A., Gaevskaya M.V., Teoriya I Prakitika Sorbtsionnykl Protsessov, 1982, 67-69). Les solutions une fois purifiées sont enfin concentrées à des teneurs comprises entre 80 et 100% en acide lactique dans l'eau et peuvent être utilisées comme matière première pour la synthèse du lactide.

Il est bien connu qu'il existe deux formes optiquement actives d'acide lactique : acide L-lactique (L-LA) et acide D-lactique (D-LA). Dès lors, le lactide, dimère cyclique de l'acide lactique, se présente sous trois formes diastéréoisomérique selon qu'ils sont constitués de deux molécules d'acide D-lactique : D-lactide (D-LD), de deux molécules d'acide L-lactique : L-lactide (L-LD), ou d'une molécule d'acide L-lactique et une molécule d'acide D-lactique : meso (D, L)-lactide. Signalons que l'on rencontre fréquemment un mélange racémique de D- et de L-lactide appelé (D,L)-lactide (D,L-LD) et caractérisé par une température de fusion (Tf = 126°C) supérieure à celle des isomères L- et D-LD purs (Tf = 97°C).

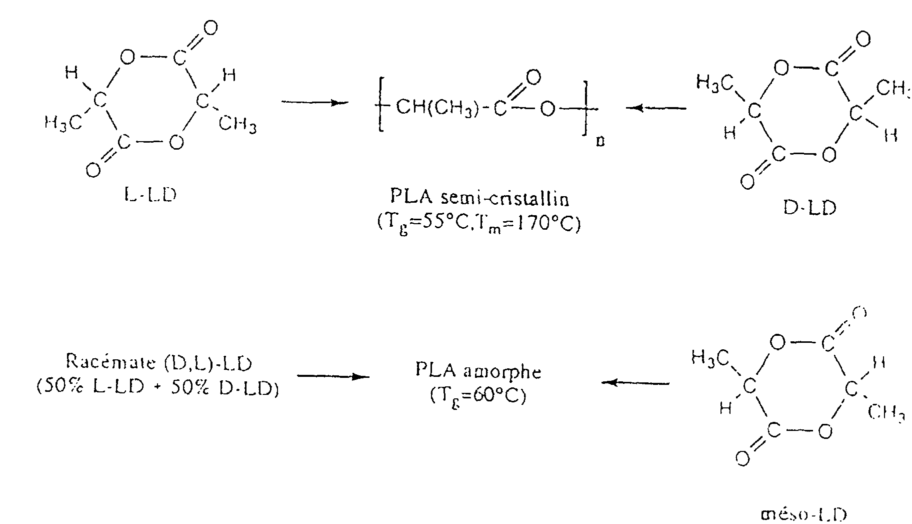

### Etat de la technique

Conformément aux pratiques antérieures, les esters cycliques souhaités étaient synthétisés en effectuant une première condensation de l'acide lactique, en un prépolymère oligomérique d'un poids moléculaire relativement important. Ce dernier étant ensuite dépolymérisé à haute température et basse pression dans un réacteur chauffé afin de former un brut d'esters cycliques. D'importants procedés de purification étaient ensuite nécessaires pour obtenir des esters cycliques d'une pureté suffisante pour permettre la synthèse de polyméres de poids moléculaire défini.

Actuellement, il existe deux grandes méthodes de production de lactide qui se distinguent essentiellement par le degré de polymérisation moyen (DP) des oligomères d'acide lactique produit lors de l'étape de condensation.

La première technique consiste à extraire l'eau d'une solution aqueuse d'acide lactique jusqu'à obtenir des oligoméres d'acide lactique (8 ≤ DP ≤ 25) Ensuite, il convient de dépolymériser ces oligomères (réaction référencée comme réaction « back-bitting ») en présence d'un catalyseur acide de Lewis, soit sous pression réduite (5 mb), à température élevée (T ≥ 230°C) (TSUYOSHI E et al, Mitsui Toatsu Chem., JP 05,105,745 ; O'BRIEN W. et al, Ecological Chemical Products Company, WO 96 06092) ou relativement élevée (T ≥ 170°C) ( MÜLLER, M. Boehringer Ingelheim KG, DE 37 08915) soit sous un flux d'azote (BATHIA K.K., et al, DuPont de Nemours, WO 92 06969 ; US 4,835,293 ; US 5,023,349) Une autre variante rapporte un procédé incluant premièrement la synthèse d'un prépolymère comprenant un polymère bloc incluant un noyau de polyéther thermiquement stable avec un hydroxyacide ou son ester polymérisé dans le noyau. Par chauffage sous vide ou flux gazeux, les extrémités de la chaîne d'hydroxyacide sont dégradées thermiquement pour former un ester cyclique pouvant être condensé sous vide (BELLIS H.E., et al, DuPont de Nemours, U.S 4,727,163, BHATIA K.K., et al, DuPont de Nemours, U S. 4,835,293).

Ce procédé se réalise dans des conditions drastiques qui, d'une part, s'avèrent coûteuses et, d'autre part, influent sur la pureté optique du lactide (pourcentage élevé de méso-LD suite à des réactions de racémisation). L'utilisation d'un réacteur de type couche-mince, particulièrement efficace dans les procédés d'évaporation, n'apporte pas d'amélioration significative (BATHIA K.K. et al., DuPont de Nemours, WO 93 02075).

La seconde méthode utilise un oligomère de DP moyen compris entre 1.5 et 2.5 pouvant être produit en phase vapeur a température élevée (200°) (BENECKE, H et al, Battelle Memorial Institute WO 92 05168) ou en phase liquide en présence d'un co-solvant formant un azéotrope avec l'eau (VERSER D.W, Biopack Technology LTD, WO 95 09142). Cet oligomère peut être cyclisé en lactide dans des conditions d'autant moins drastique qu'un solvant aromatique est utilisé pour extraire le lactide du milieu réactionnel (T° ≈ 200°C, P ≈ 10-100mbars) (SINCLAIR R.G. et al, Battelle Memorial Institute, WO 92 05167 ; BENECKE, H.P., Biopack Technology LTD, WO 93 19058). Les principaux inconvénients de ce procédé résident dans la présence d'un solvant, souvent aromatique, de haut point d'ébullition, d'une température de réaction > à 180°C, de son manque de sélectivité et de sa quantité non négligable d'impuretés protiques (surtout carboxylique).

De manière générale, le brut obtenu suite a ces diverses voies de synthèses contient toute une série d'impuretés protiques (acides carboxyliques, composés hydroxylés, eau, ...) qu'il faudra extraire afin d'obtenir une pureté suffisante pour pouvoir l'intégrer au procédé de polymérisation par ouverture de cycle.
Parmi les procédés utilisés et connus de l'homme de l'art, on retiendra soit une recristallisation en solvant organique ( BENECKE, H.P., Biopack Technology LTD, WO 93 19058 ; BATHIA K.K. et al., DuPont de Nemours, WO 93 15069) ou aqueux (YOMAGUCHI et al, Musashino Chemical Laboratory, US 5,502,215), soit une distillation avec extraction intermédiaire (NEVILLE E., et al, DuPont de Nemours, US 5,236,560 ; Gruber P.R., Cargill Inc. US 5,247,059), soit une recristallisation en milieu fondu (Fridman, I.D. et al, Camelot Technologies Inc., US 5,264,592) soit une succession de deux ou plusieurs procédés énoncés ci-dessus ( O'Brien. W. et al, Ecological Chemical Products Company, WO 96 06092. Gruber P.R., Cargill Inc. WO 95 09879).

### Brève description de l'invention

La présente invention consiste en un procédé de production d'esters cycliques dimériques, en particulier du lactide et du glycolide, au depart d'un «brut d'estérifïcation partielle» tel que décrit dans les revendications.

Dans la suite de la présente demande, toutes les descriptions relatives au lactide peuvent s'appliquer au glycolide sauf en matière de stéréochimie puisque l'acide glycolique ne présente pas d'isomères optiques.

Le brut d'estérification comporte un mélange d'acide lactique et/ou glycolique (monomère), (de préférence en composé minoritaire), et d'un ester lactique et/ou glycolique (LR), (de préférence composé majoritaire). Ce brut d'estérification peut-être obtenu soit par simple mélange des produits purs, soit au départ d'acide lactique et/ou glycolique ou de ses dérivés issus d'un procédé de fermentation et éventuellement purifiés par toute technique connue de l'homme de l'art telle par exemple l'extraction liquide-liquide. Le dérivé d'acide lactique en solution aqueuse ou organique est estérifié par un alcool. Une technique privilégiée serait de produire simultanément l'alcool et l'acide lactique et/ou glycolique par hétérofermentation ou fermentation mixte.

En ce qui concerne l'obtention du mélange de départ, il est fait référence à toute méthode connue de l'homme de l'art et décrite ci-avant. Le procédé de synthèse décrit dans cette invention a l'avantage de permettre l'obtention d'un lactide de très haute qualité avec un rendement massique élevé et une consommation énergétique minimale. On entend par très haute qualité, une pureté suffisante pour pouvoir être utilisée comme monomère pour la synthèse du PLA par ouverture de cycle. Suivant la voie de purification suivie, deux qualités d'ester cyclique dimérique peuvent être obtenues dans le cadre de cette invention. La première permet de fournir un lactide d'une très grande pureté chimique mais non optique, ce qui réduira son champ d'application potentiel mais qui reste néanmoins intéressante car la teneur en méso-lactide résiduel est inférieure à 2%. La seconde, par contre, délivre un produit d'une grande pureté chimique et optique, ce qui permet de généraliser son application.

Les aspects quantitatif et sélectif de ce procédé de synthèse sont assurés avantageusement par la mise en oeuvre conjointe (1) d'une dimérisation douce et sélective de l'ester lactique en favorisant la réaction de condensation entre la fonction hydroxyle de l'ester et la fonction acide carboxylique de l'acide, (2) d'une cyclisation douce et sélective de l'ester dimérise en lactide en favorisant les reactions de transesterification intramoléculaires, et (3) de la purification du brut obtenu soit (a) par une double distillation , soit (b1) par recristallisation du LD à température ambiante dans un solvant qui peut être celui utilisé lors de l'étape de cyclisation, soit (b2) par une distillation couplée à une recristallisation en milieu fondu.

L'aspect énergétique minimal est quant à lui atteint suite aux conditions de températures et de temps de séjour relativement faibles nécessaires pour les étapes de dimérisation de l'ester et de cyclisation de l'ester dimérisé en lactide et/ ou glycolide.

De manière préférée, le mélange de départ correspond à une composition relative en ester d'acide lactique et/ou glycolique et en acide lactique et/ou glycolique comprise entre 50 et 99 % en ester et préférablement entre 70 et 95 %. Cette mixture que nous appellerons « brut d'estérification » peut-être obtenue par mélange des produits purs, mais une approche privilégiée de la présente invention consiste à extraire le mélange à un moment bien déterminé de l'étape d'estérification.

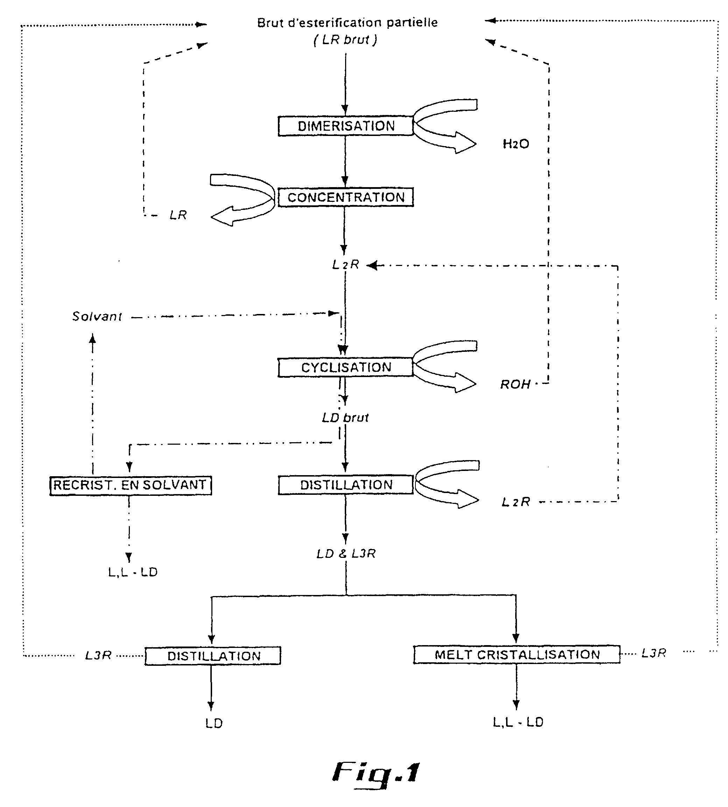

La figure 1 illustre le procédé de synthèse du lactide tel qu'il est fait référence dans la présente invention.
Ce procédé comprend essentiellement les trois étapes suivantes :

### (1) une dimérisation de l'ester lactique

La première étape (dimérisation) considérée dans le cadre de l'invention consiste en une dimérisation, de préférence douce et sélective de l'ester lactique en favorisant la réaction de condensation entre la fonction hydroxyle de l'ester et la fonction acide carboxylique de l'acide au détriment des réactions d'hydrolyse (rupture du lien ester par l'eau) et de transestérification intermoléculaire (oligomérisation de l'ester lactique).

Avantageusement, cette étape de dimérisation est réalisée d'une part, dans des conditions réactionnelles très douces et un temps de séjour relativement court, ce qui contrairement aux procédés en vigueur actuellement, suffisent à initier et compléter la reaction de dimerisation. En effet, une approche privilegiée de la présente invention considére que des températures réactionnelles n'excédant pas 190°C, préférablement inférieures à 175°C, plus préférablement inférieures à 165°C et plus préférablement encore inférieures à 150°C, et des temps de séjour compris préférablement entre 0.1 et 60 min, plus préférablement entre 0.1 et 30 min, plus préférablement encore compris entre 0.1 et 15 min, et plus préférablement encore compris entre 0.1 et 5 min suffisent pour initier et compléter la réaction, ce qui élimine tout problème de racémisation et réduit drastiquement les dépenses énergétiques.

D'autre part, grâce au choix judicieux du mélange de départ, du catalyseur, du type de réacteur et des conditions réactionnelles, la sélectivité liée à cette étape est très élevée. On entend par sélectivité élevée dans le cadre de cette étape de l'invention, une réactivité totale vis-à-vis de l'acide lactique (LA) et/ou de l'acide glycolique (acidité résiduelle due à LA nulle), et une teneur en trimère et tétramère (seuls oligomères présents) de l'ester inférieure à 10%, préférablement inférieure à 5% et plus préférablement encore inférieure à 2%.

Un très grand nombre de catalyseur d'estérification pourraient être utilisés dans le cadre de cette invention pour la formation de lactides. Il y a entre autres les catalyseurs acides, solides et hétérogènes tels que les aluminiques, les eta-, theta-, delta- et gamma aluminiques, l'yttrium, la silice, le trioxyde d'antimoine, l'oxyde de plomb le béryllium, le sulfate d'aluminium; les catalyseurs à base de sel organique de métal tels que l'octoate d'étain; les catalyseurs acides et solubles tels l'acide sulfurique, l'acide méthanosulfonique, l'acide paratoluène sulfonique, l'acide trifluorométhane sulfonique et l'acide toluènesulfonique-titane; les catalyseurs micéllaires tels que les sels de sulfosuccinates ; les catalyseurs acides échangeurs d'ions; les phosphates , les catalyseurs à base de silice tels que les amino-silicates, les enzymes tels que les hydrolases, les zéolites, les catalyseurs non polaires tels que le polyoxyéthylène nonyl phénol; les sels tel que le chlorure de cuivre. Dans une approche privilégiée de la présente invention, les acides de Brönsted, nécessitant une faible énergie d'activation et favorisant les réactions d'estérification plutôt que de transestérification, seront utilisés comme catalyseur de dimérisation et plus particulierement l'acide para-toluène sulfonique (APTS). Les teneurs en catalyseur préconisées sont tributaires de la nature du catalyseur, de la composition du mélange initial et des conditions réactionnelles mais restent toutefois, dans le cadre de cette invention, comprises entre 0 et 5% et préférablement comprises entre 0.1 et 2%.

Dans la présente invention, la configuration du réacteur joue un rôle afin de maintenir une sélectivité élevée pour la réaction de dimérisation et d'éviter la formation d'oligomères de l'ester lactique et/ou glycolique. Le réacteur pourra avantageusement être conçu pour pouvoir offrir, d'une part, une capacité de volatilisation importante afin d'éliminer l'eau produite (et une partie de l'ester de départ en excès) et déplacer l'équilibre vers la formation du dimère, d'autre part, un temps de réaction suffisant pour qu'elle soit complète, et enfin un temps de séjour non excessif pour éviter les réactions d'oligomérisation. Dès lors, dans le cadre de cette invention, tout réacteur susceptible de remplir ces contraintes telle que par exemple la famille des réacteurs couche mince peuvent avantageusement être utilisés. De plus, plusieurs technologies connues de l'homme de l'art peuvent être envisagées, dans le cadre de cette invention, afin de favoriser la vaporisation et l'extraction de l'eau du milieu réactionnel, comme par exemple : entraînement par un flux d'azote, par un système de vide, ...

La vitesse de la réaction de dimérisation est d'autant plus importante que la fraction molaire en acide dans le mélange de départ est importante. Cependant, une fraction molaire trop élevée en acide est à éviter car elle favorise les réactions de polycondensation (formation d'oligomères d'acide) qui nuisent à la sélectivité recherchée dans le cadre de cette invention. Il est dès lors possible, en fonction de l'efficacité du réacteur utilisé, qu'une étape de concentration supplémentaire soit nécessaire afin d'extraire l'ester lactique et/ou glycolique « monomèrique » (LR) en excès. De même, lors de cette étape de concentration, nous veillerons à utiliser un réacteur qui permettra, dans des conditions très douces, l'extraction du monomère sans pour autant induire les réactions de transestérifications intermoléculaires. Dès lors, dans le cadre de cette invention, tout réacteur, susceptible de remplir ces contraintes et, connu de l'homme de l'art peut être tout à fait approprié (par exemple la famille des réacteurs couche mince).

### (2) une cyclisation de l'ester dimérise en lactide et/ou glycolide

Cette étape de l'invention consiste au départ de l'ester dimérisé obtenu en (1) et caractérisé par une composition relative en ester dimérisé comprise entre 50 et 100% et préférablement comprise entre 70 et 100%, et une teneur en trimère et tétramère (seuls oligomères présents de l'ester lactique) inférieure à 15 %, préférablement inférieure à 10%, plus préférablement inférieure à 5% et plus préférablement encore inférieure à 2%, en une cyclisation sélective en lactide et/ou glycolide favorisant les réactions de transestérifications intramoléculaires au détriment des réactions intermoléculaires telles que racémisation (générant du méso-LD), ouverture de cycle ou transestérifications (générant des oligomères de degré de polymérisation supérieur à deux).

Dans le cadre de cette invention, nous avons clairement démontré qu'il s'agit d'un processus de cyclisation d'un dimére linéaire et non d'un processus de dépolymérisation d'oligomères d'acide lactique et/ou glycolique. En effet, les autres procédés, connus de l'état de l'art actuel et revendiquant une cyclisation du dimère cyclique, effectuent la synthèse au départ d'un mélange réactionnel constitué en permanence d'environ 10 à 20% d'oligomères d'acide lactique allant jusqu'à un degré de polymérisation (DP) égal à 5, ce qui laisse une certaine incertitude quant au mécanisme réactionnel réel. Or, une des approches privilégiées de la présente invention a clairement démontré un mécanisme réactionnel exclusivement basé sur une cyclisation du dimère linéaire. En effet, dans le cadre des expériences (reprises dans l'exemple 7) on remarque que le contenu en lactyl lactate d'éthyle décroît au fur et à mesure de la formation de lactide, la composition de tout autre composant restant inchangée etant donné la grande sélectivité du procédé.

Cette étape permet avantageusement de synthetiser l'ester cyclique dimérique dans des conditions extrêmement douces jamais rencontrées dans les procédés conventionnels. En effet, une approche privilégiée de la présente invention considère que des températures réactionnelles n'excédant pas 190°C et preférablement inférieures à 175°C et plus préférablement encore inférieures à 165°C suffisent à l'étape de cyclisation du dimère linéaire. Ceci permet avantageusement de réduire les coûts énergétiques, d'investissements dans des appareillages coûteux, mais aussi de limiter la formation de sous-produits ou impuretés qu'il est nécessaire d'éliminer dans une étape ultérieure de purification. Dans le cadre de la présente invention, la purification consiste essentiellement en la séparation de l'ester cyclique dimérique du dimère linéaire, tandis que la quantité de trimère et tétramère linéaires est inférieure à 10% et préférablement inférieure à 5% et plus préférablement encore inférieure à 2%.

Une approche privilégiée de la présente invention pour favoriser la sélectivité et donc la cyclisation au détriment de la polymérisation, consiste à favoriser les réactions intramoléculaires plutôt que les réactions intermoléculaires des molécules de dimère linéaire. Toute technique connue de l'homme de l'art visant cet objectif est envisageable et, préférablement, dans le cadre de cette invention, effectuer la réaction en solution dans un solvant de préférence inerte susceptible de recristalliser l'ester cyclique dimérique à froid, ou effectuer la réaction en phase vapeur avec ou sans gaz vecteur (gaz inerte N2,.. ). Les solvants susceptibles d'être utilisés et connus de l'homme de l'art, sont des cétones, des éthers, des solvants aromatiques, aliphatiques, des solvants à base de silicone et des solvants halogénés comme par exemple : acétone, 2-butanone, 2-pentanone, 2-hexanone, 2-heptanone, anisole, éthyl éther, isopropyl éther, butyl éther, méthyl phényl éther, benzène, cumène, p-cymèné, p-xylène, o-xylène, m-xylène, toluène, cyclohexane, hexane, heptane, octane, nonane, 1-pentene, 2-octanone, 4-méthyl anisole, 1,2-diméthoxybenzène, 1,3-diméthoxybenzène, 1,4-diméthoxybenzène, mésitylène, chlorobenzène, 1,2-dichlorobenzène, 1,3-dichlorobenzène, 1,4-dichlorobenzène, 2-chlorotoluène, 3-chlorotoluène et 4-chlorotoluène.

La majorité des catalyseurs d'estérification énumérés dans le point (1) pourraient être utilisés pour cette seconde étape de cyclisation. Cependant, dans le cadre de cette invention, nous retiendrons de préférence tous les catalyseurs ne favorisant pas la synthèse du polymère d'acide lactique (PLA) au départ de lactide comme il est bien connu dans l'état de l'art de la polymérisation par ouverture de cycle. Dans une approche privilégiée de la présente invention, l'acide para-toluène sulfonique (APTS) et le chlorure de cuivre (CuCl) se sont avérés particulièrement efficaces et sélectifs. Les teneurs en catalyseur préconisées sont tributaires de la nature du catalyseur, de la composition du mélange initial et des conditions réactionnelles mais restent toutefois, dans le cadre de cette invention, préférablement comprises entre 0 et 5% et plus préférablement comprises entre 0.1 et 2%.

La réaction de cyclisation est typiquement plus lente que la réaction de dimérisation. En effet, cette dimérisation est une réaction de condensation entre une fonction acide carboxylique et un groupe hydroxyle libérant de l'eau, tandis que la réaction de cyclisation fait intervenir une réaction entre un groupe ester éthylique, moins réactionnel qu'un acide carboxylique, et une fonction hydroxyle. Dès lors, un simple réacteur de type batch sous un flux d'azote ou tout autre réacteur connu de l'homme de l'art susceptible de faciliter l'extraction de l'alcool produit et permettant un temps de séjour compris préférablement entre 0.1 et 120 min, plus préférablement entre 0.1 et 60 min et plus préférablement encore compris entre 0.1 et 30 min, pourra être envisagé dans le cadre de cette invention.

### (3) la purification du brut obtenu

Cette étape de l'invention consiste, au départ d'un ester cyclique dimérique brut (lactide ou glycolide impur) obtenu en (2) et de préférence caractérisé par une composition relative en monomère et dimère linéaire comprise entre 0 et 80%, plus préférablement comprise entre 10 et 60%, et plus préférablement encore comprise entre 10 et 40%, une teneur en méso-LD comprise préférablement entre 0 et 5%, plus préférablement comprise entre 0 et 2% et plus préférablement encore inférieure à 1%, une teneur en trimère et tétramère (seuls oligoméres présents de l'ester lactique) préférablement inférieure à 15%, plus préférablement inférieur à 10%, plus préférablement encore inférieure à 5% et plus préférablement encore inférieure à 2%, le solde étant constitué de L-LD, en une purification par élimination des impuretés protiques afin d'obtenir un lactide de qualité suffisante pour pouvoir être utilisé comme monomère pour la synthèse du PLA par ouverture de cycle.

Suivant la voie de purification choisie, deux qualités de lactide peuvent être obtenues dans le cadre de cette invention. La première, (a) caractérisée par une double distillation. permet de fournir un lactide d'une trés grande pureté chimique mais ne sera pas pur optiquement. En effet, bien que le procédé de cyclisation ne génère que très peu de méso-lactide, il ne pourra être éliminé car le procédé de distillation n'est pas stéréospécifique. Le lactide obtenu pourra néanmoins être utilisé en polymérisation (acidité, teneur en eau faible), car la teneur en méso-lactide reste faible (<2% voire <1%). Bien que les propriétés mécaniques du PLA sont fortement tributaires de la teneur en méso-lactide du monomère, celui-ci pourra être utilisé pour des applications spécifiques.

Cette première voie (a), consiste à alimenter le mélange gazeux issu du milieu réactionnel dans une première colonne de distillation qui permettra la récupération des composants les plus volatiles (LR voire L2R) en tète de colonne. Les composés lourds, à savoir le lactide et les esters de l'acide lactique (L2R, L3R & L4R), sont ensuite alimentés dans une seconde colonne qui permettra de récupérer en tête un lactide pur.

Une approche privilégiée de la présente invention, considère le cas de l'ester éthylique de l'acide lactique. En effet, lors de la première distillation, le lactate d'éthyle et le dimère linéaire sortiront en tête de colonne et pourront être intégralement recyclés au niveau de l'étape de cyclisation. Une purge pourra toutefois être instaurée afin d'éviter une concentration trop importante en ester « monomérique ». Cette purge n'en est pas une en réalité, car le produit extrait pourra être ré-injecté au niveau de l'étape d'estérification. De plus, étant donné la différence relativement importante existant entre les courbes de tension de vapeur du LD et du L3Et, cette seconde séparation ne requiert pas un grand nombre de plateaux théoriques et, à la limite, un simple procédé flash pourrait être envisagé. Il pourrait même être envisagé d'incorporer le procédé flash avec extraction sous forme vapeur au niveau du ballon de distillation de la première colonne et donc d'éviter la complexité liée au pilotage de deux unités contiguës.

La seconde voie (b), par contre, délivre un produit d'une grande pureté chimique et optique, ce qui permet de généraliser son application pour la polymérisation. Dans le cadre de cette seconde voie, deux possibilités sont envisagées suivant que l'étape de cyclisation est menée dans un solvant ou en phase vapeur.
L'une des deux possibilités (b1) consiste à sélectionner correctement le solvant destiné à favoriser les réactions intramoléculaires et à diminuer la probabilité de réactions intermoléculaires. En effet, le solvant devra avantageusement être caractérisé par une température d'ébullition suffisamment élevée afin d'éviter la limitation de la température de cyclisation, par sa capacité de solubiliser le L-LD à température réactionnelle et de l'insolubiliser à température ambiante et, par sa sélectivité vis-à-vis du méso-LD. Le L-LD est ensuite séparé par filtration et le solvant est extrait par toute technique connue de l'homme de l'art, comme par exemple l'évaporation, l'extraction,... Les solvants susceptibles d'être utilisés sont des cétones, des éthers, des solvants aromatiques, aliphatiques, des solvants à base de silicone et des solvants halogénés comme précédemment cités.

L'autre possibilité (b2), est proche de celle décrite en (a) mais sera adaptée afin d'assurer la stéréospécificité du procédé. En effet, étant donné la faible teneur en esters lourds (<5% voire < 2% en L3Et&L4Et), la seconde colonne de distillation, suivant des considérations économiques, peut-être soit maintenue et un procédé de recristallisation en milieu fondu ajouté, soit substitué par un procédé flash incorporé ou non dans la première colonne et par un procédé de recristallisation en milieu fondu ou soit simplement par un procédé de recristallisation en milieu fondu. Le produit traité par cette technologie subit un refroidissement contrôlé qui va initier la cristallisation. Durant cette étape, les impuretés sont concentrées dans la phase liquide entourant les cristaux. Une fois la cristallisation terminée, la phase liquide est extraite du cristallisoir par gravité, ce qui laisse un cristal enrobé d'un film chargé d'impuretés. Afin d'éliminer ce film, une fonte partielle des cristaux est opérée. Le produit liquide ainsi obtenu se mélange avec le film et est évacué par gravité hors du cristallisoir. Finalement, lorsque la pureté souhaitée est atteinte, le contenu du cristallisoir est fondu et récupéré.

### Description détaillée de l'invention

D'autres détails et particularités de l'invention, donnés ci-après à titre d'exemples non-limitatifs, ressortent de la description et de la figure 1 comme quelques formes possibles de réalisation.

### Exemples

Sauf indications contraires, tous les pourcentages relatifs aux catalyseurs sont des pourcentages en poids, et ceux relatifs aux compositions et aux fractions sont des pourcentages en moles.

### Exemple 1 : Dimérisation douce et sélective de l'ester lactique

Un excès de lactate d'éthyle commercial a été mis en présence d'acide lactique frais se présentant essentiellement sous forme monomérique (qualité « heat stable ») de sorte à reconstituer un « brut d'estérification » d'une composition relative en ester lactique de 90%.
Le mélange est introduit dans un ballon à 3 tubulures de 250 ml surmonté d'une colonne de reflux maintenue à 115°C, suivie d'un coude de distillation, d'un thermomètre, d'un réfrigérant refroidi à l'eau de ville et d'un ballon récepteur. Le ballon à 3 tubulures est encore équipé d'un tuyau en verre terminé par un fritté, d'une sonde thermique Pt 100 connectée à un régulateur qui contrôle la puissance délivrée à la calotte chauffante placée sous ce ballon. De manière à homogénéiser le milieu réactionnel et à favoriser l'évaporation des produits de réaction, un flux d'azote gazeux est passé à travers le fritté. La température dans la colonne de reflux est gerée par un bain thermostatique. L'entièreté de l'appareil est calorifugé. Le temps tₒ est compté à partir du moment où la sonde thermique atteint la température de consigne de réaction.

**Tableau 1:**

| *Cinétique de formation de L2Et à 145°C, sous flux d'azote et en présence de 1%pds d'APTS (température colonne de reflux : 115°C. LA : 100%pds et 89.5%pds d'acidité libre,et LEt : d'acidité libre négligeable).* | | | | | | | | | | |
|---|---|---|---|---|---|---|---|---|---|---|
| | | | | | | GC normalisée en fonction de la F_{LA} | | | | |
| F_{LA} (%) | T (min) | Acid. (mmol/ g) | M th distil (g) | M distil (g) | Ttête reflux (°C) | LEt | L2Et | L3Et | L4Et | LD |
| 9.89 | 0 | 0.9 | 3.8 | 0.0 | 106 | 90.1 | 0.0 | 0.0 | 0.0 | 0.0 |
| 9.89 | 5 | <0.1 | 3.8 | 7.1 | 102 | 72.1 | 17.1 | 0.9 | 0.0 | 0.0 |
| 9.89 | 15 | <0.1 | 3.8 | 15.0 | 88 | 67.4 | 20.3 | 2.4 | 0.0 | 0.0 |
| 9.89 | 30 | <0.1 | 3.8 | 25.5 | 97 | 56.1 | 28.7 | 5.3 | 0.0 | 0.0 |
| 9.89 | 45 | ND | 3.8 | 33.8 | 95 | 48.6 | 32.7 | 8.3 | 0.5 | 0.0 |
| 9.89 | 60 | ND | 3.8 | 48.7 | 100 | 42.5 | 33.1 | 12.4 | 1.7 | 0. |
| 9.89 | 90 | ND | 3 8 | 83.8 | 100 | 19.4 | 34.2 | 26.2 | 7.0 | 3.6 |
| 9.89 | 120 | ND | 3.8 | 99.8 | 78 | 7.9 | 19.6 | 36.8 | 17.2 | 9.5 |
| ND : pas déterminé | | | | | | | | | | |
| F_{LA} : fraction molaire en acide lactique | | | | | | | | | | |
| Mth : masse théorique de distillation | | | | | | | | | | |
| M : masse du distillat | | | | | | | | | | |
| Ttête : température de tête du reflux | | | | | | | | | | |

On remarque, dès les premières minutes de réaction, que l'acidité libre est pratiquement nulle, ce qui signifie que l'acide lactique a entièrement réagi, et que le L2Et est synthétisé sélectivement. Parmi les distillats, une analyse GC a permis de mettre en évidence la présence concomitante de LEt, d'EtOH et d'eau. La masse distillée est toujours largement supérieure à celle calculée sur base d'une réaction quantitative et de la formation exclusive d'eau. Entrainement important de lactate d'éthyle n'est cependant pas un problème, mais plutôt une aubaine, dans la mesure où l'objectif est d'obtenir sélectivement du lactyllactate d'éthyle La formation de lactide et d'oligomères de masse moléculaire supérieure à celle du L2Et confirme qu'au-delà des réactions de condensation, des réactions de transestérification inter- et intramoléculaires se produisent. Enfin, il est remarquable de constater qu'après 2 heures de réaction à 145°C, il y a déjà formation de 10% de LD.

### Exemple 2 : Concentration sélective de l'ester dimérisé

Un mélange issu de l'étape de dimérisation est alimenté en continu dans un évaporateur en verre borosilicate, à film mince mécaniquement agité avec condenseur interne (short-path) de marque UIC présentant des surfaces de chauffage et de condensation de 0.06 m², précédé d'un dégazeur-préchauffeur dont la température est ajustée par circulation d'huile caloporteuse. Les résultats présentés au tableau 2 ont été obtenus avec une température de dégazage de 60°C, une température de condenseur de 15°C, une pression de 2 mbars absolus, une vitesse de rotation du rotor de 300 rpm (rotation par minute) et un débit d'alimentation de 550 g/h, et montrent clairement la concentration sélective du dimère linéaire, en effet l'accroissement en L3Et n'est dû qu'à un effet de concentration.

**Tableau 2:**

| *Composition des condensats (Nc) et distillats (Nd) de L2Et lors d'une concentration dans un évaporateur de type couche mince avec condenseur interne. Les valeurs GC ne sont pas corrigées en fonction de la fraction molaire initiale en acide lactique, soit 10.0%mo.* | | | | | | | | |
|---|---|---|---|---|---|---|---|---|
| | | | | GC (%) | | | | |
| Ech. | Mₒ(g) | M_{f}(g) | Acid. (mmol/g) | LEt | L2Et | L3Et | L4Et | L5Et |
| N 0 | 1639.9 | - | 0.04 | 78.8 | 19.4 | 1.9 | 0.0 | 0.0 |
| Nd | - | 1040 1 | ND | 99.2 | 0 | 0.0 | 0.0 | 0.0 |
| Nc | - | 599 8 | 0.08 | 14.3 | 76.8 | 8.9 | 0.0 | 0.0 |
| Mₒ : Masse de depart | | | | | | | | |
| M_{f} : Masse finale | | | | | | | | |
| Nₒ : alimentation | | | | | | | | |

### Exemple 3 : Influence de la nature du catalyseur sur la réaction de cyclisation de l'ester dimérisé

La réaction de cyclisation du lactyllactate d'éthyle en lactide a été réalisée dans un réacteur de type batch semblable à celui utilisé pour l'étape de dimérisation. La réaction a été réalisée a pression atmosphérique avec différents catalyseurs présents à raison de 1 %pds et à différentes températures. Le tableau 7 présente les résultats obtenus pour différents catalyseurs : acide paratoluène sulfonique (APTS), bis 2-ethylhexanotate d'étain (II) (Sn(Oct)2), oxide de zinc (II) (ZnO) et chlorure de cuivre (I) (CuCl).

**Tableau 3:**

| *Influence de la nature du catalyseur (1%pds) sur la réaction de cyclisation en masse du lactyllactate d'éthyle dans un réacteur de type batch et sous flux d'azote. La composition du mélange de départ est la suivante, exprimée en pourcent et déterminée par GC : LEt 5.4, L2Et 77.1, L3Et 16.2, L4Et 1.0, L5Et 0.3.* | | | | | | | | | | |
|---|---|---|---|---|---|---|---|---|---|---|
| Catalyseur | t (min) | GC normalisé en fonction des distillats | | | | | | | PLA^{b} | Acid. (mmol /g) |
| | | LEt | m-LD | L.L-LD | L2Et | L3Et | L4Et | L5Et | | |
| APTSd | 6 | 3.6 | 0.0 | 1.7 | 24.6 | 27.0 | 20.9 | 5.8 | - | ND |
| | 15 | 1.5 | 0.0 | 4.2 | 15.8 | 23.4 | 27.5 | 8.8 | - | 0.25 |
| Sn(Oct)2d | 15 | 8.9 | 1.1 | 5.2 | 28.5 | 28.2 | 14.8 | 1.1 | - | 0.17 |
| | 25 | 6.8 | 1.0 | 5.4 | 23.1 | 25.7 | 18.4 | 3.5 | + | ND |
| | 30 | 1.9 | 1.2 | 4.3 | 18.8 | 27.1 | 19.4 | 5.6 | + | ND |
| ZnO^{a} | 30 | 0.0 | 0.1 | 6.8 | 21.5 | 27.7 | 16.7 | 4.7 | + | 0.47 |
| | 60 | 0.0 | 0.0 | 4.8 | 5.5 | 28.4 | 23.7 | 5.7 | + | ND |
| CuCl^{a}^{,}^{c} | 5 | 3.8 | 0.0 | 6.1 | 18.5 | 27.0 | 25.5 | 6.7 | - | ND |
| | 15 | 2.3 | 0.0 | (2.9) | 21.3 | 26.6 | 25.4 | 6.7 | - | 0.18 |
| | 25 | 1.6 | 0.0 | 4.8 | 16.0 | 26.7 | 25.8 | 8.2 | - | ND |

| | | | | | | | | | | |
|---|---|---|---|---|---|---|---|---|---|---|
| ^{a}- Au delà du dernier temps t présenté, le milieu devient trop visqueux pour assurer une agitation convenable et analyser les échantillons. | | | | | | | | | | |
| ^{b}-La présence de PLA est détectée par l'apparition de particules blanches insolubles dans le milieu. | | | | | | | | | | |
| ^{c}-La composition du lactyllactate d'éthyle de départ est légèrement différente, soit : LEt 7.9, L2Et 77.7, L3Et 12.2, L4Et 1.6, L5Et 0.1 ND : non déterminé. | | | | | | | | | | |

Il découle de ces résultats que le lactyllactate d'éthyle est consommé quel que soit le catalyseur, même si la réaction est relativement lente à ces faibles températures. La proportion de LEt diminue également au cours du temps, tandis que des oligomères de masse moléculaire supérieure au dimère sont formés. Aucun catalyseur ne se dégage vraiment du lot par une production élevée de lactide. En présence de Sn(Oct)2 et de ZnO, connus pour catalyser la polymérisation par ouverture de cycle du lactide, il y a rapidement formation de chaînes polyméres insolubles dans le milieu a ces températures.

En d'autres termes, il existe une compétition entre la réaction de formation du lactide et la polymérisation du lactide par ouverture de cycle et ceci dés sa formation. Ce comportement témoigne de la nécessité de favoriser la cyclisation au détriment de la polymérisation. Dans ce but, deux voies, qui consistent à favoriser les réactions intramoléculaires (cyclisation) au détriment des réactions intermoléculaires (polymérisation et transestérification), sont envisageables, soit effectuer la réaction en solution dans un solvant inerte, soit effectuer la réaction à l'état de vapeur, c'est-à-dire des conditions où on favorise les réactions intermoléculaires plutôt que les réactions intramoléculaires.

### Exemple 4 : Influence de la température sur la réaction de la cyclisation de l'ester dimérisé

Dans un réacteur semblable à celui utilisé dans l'exemple 2, deux catalyseurs symbolisant deux types de catalyse acide, ont été utilisés pour étudier l'influence de la température de réaction, à savoir l'APTS : acide de Brönsted, et le Sn(Oct)2 : acide de Lewis (Tableau 4).
En ce qui concerne l'influence de la température sur la réaction de cyclisation catalysée par Sn(Oct)2, la formation de lactide est la plus importante à 145°C après 25 min, soit 5.4%. Au-delà de 25 min, la réaction prépondérante devient l'ouverture des cycles lactides ce qui se note par une diminution de la proportion de LEt et de lactide, et une augmentation de la fraction d'oligomères de masse moléculaire supérieure au dimére. A 80°C, moins de réaction de polymérisation ou de transestérification intermoléculaire se produisent mais l'effet escompté n'a pas été observé, à savoir la cristallisation du lactide au cours de sa formation. De maniére générale, on constate que Sn(Oct)2 induit des réactions de racémisation générant du méso-lactide, même aux températures les plus basses. Par ailleurs, il y a formation de polylactide dès 145°C. L'APTS est un catalyseur plus sélectif dans la mesure où il n'y a pas formation de chaînes polymères et où les réactions de racémisation sont moins nombreuses. La production de lactide est minime, excepté à 180°C où elle atteint 13.1 %, tandis que le LEt a été largement volatilisé.

**Tableau 4:**

| *Influence de la température sur la réaction de cyclisation en masse du lactyllactate d'éthyle dans un réacteur de type batch et sous flux d'azote : A- Sn(Oct)2 et B- APTS. Le lactyllactate d'éthyle de départ est soit Gc 2, Pc 2 ou Ncl dont les compositions sont reprises dans le tableau*. | | | | | | | | | | | | | |
|---|---|---|---|---|---|---|---|---|---|---|---|---|---|
| T (°C) | Type de catalyseur | M catalyseur (%pds) | L2Et₀ | t | GC nonnalisé en fonction des distillats | | | | | | | PLA (min) | Acide. (mmol/ g) |
| | | | | | LEt | m-LD | L.L-LD | L2Et | L3Et | L4Et | L5Et | | |
| 80 | A | 1 | Pc2 | 90 | 3.0 | 0.0 | 3.0 | 49.1 | 30.7 | 9.0 | 0.0 | - | 0.07 |
| | | | | 120 | 2.5 | 1.7 | 0.8 | 40.9 | 34.1 | 15.7 | 1.7 | - | ND |
| 145 | A | 1 | Gc2 | 15 | 8.9 | 1.1 | 5.2 | 28.5 | 28.2 | 14.8 | 1.1 | + | 0.17 |
| | | | | 25 | 6.8 | 1.0 | 5.4 | 23.1 | 25.7 | 18.4 | 3.5 | + | ND |
| | | | | 30 | 1.9 | 1.2 | 4.3 | 18.8 | 27.1 | 19.4 | 5.6 | + | ND |
| 145 | B | 1 | Gc 2 | 6 | 3.6 | 0.0 | 1.7 | 24.6 | 27.0 | 20.9 | 5.8 | - | ND |
| | | | | 15 | 1.5 | 0.0 | 1.9 | 15.8 | 23.4 | 27.5 | 8.8 | - | 0.25 |
| 180 | B | 1 | Nc 1 | 15 | 1.7 | 0.6 | 13.1 | 9.0 | 28.3 | 13.5 | 3.7 | - | ND |
| Pc2 : LEt 7.9, L2Et 77.7, L3Et 12.2, L4Et 1.6, 15Et 0 .1 | | | | | | | | | | | | | |
| Gc2: LEt 5.4, L2Et 77.0, L3Et 16.2, L4Et 1.0, L5Et 0.3 | | | | | | | | | | | | | |
| Nc1: LEt 14.3, 1.2Et 76.8, L3Et 8.9 | | | | | | | | | | | | | |

### Exemple 5 : Influence de la quantité de catalyseur sur la réaction de la cyclisation de l'ester dimérisé

Pour les catalyseurs ayant été reconnus plus selectifs, à savoir : l'APTS et le CuCl, nous avons envisagé d'augmenter la proportion de catalyseur. Des résultats obtenus et répertoriés dans le tableau 5, on observe qu' augmenter la proportion d'APTS de 1 à 2 % en poids semble accélérer la réaction de cyclisation du lactate d'éthyle en lactide, c'est à dire les réactions intramoléculaires au détriment des réactions intermoléculaires de transestérification. Par contre, pour une quantité d'APTS de 5 % en poids, aucun effet cinétique substantiel ne peut être détecté. Lorsque le chlorure de cuivre (I) est utilisé comme catalyseur, la quantité de catalyseur ne semble pas avoir d'effet notable sur la cinétique de la reaction de cyclisation.

**Tableau 5:**

| *Influence de la quantité de catalyseur sur la réaction de cyclisation en masse du lactyllactate d'éthyle (LEt 14.3, L2Et 76.8, L3Et 8.9.) dans un réacteur de type batch, sous flux d'azote et à 180°C.* | | | | | | | | | | | |
|---|---|---|---|---|---|---|---|---|---|---|---|
| Cata | M cata (% pds) | t (min) | GC normalisé en fonction des distillats (%) | | | | | | | PLA | Acid (mmol /g) |
| | | | LEt | m-LD | L.L-LD | L2Et | L3Et | L4Et | L5Et | | |
| APTS | 1 | 2 | 8.6 | 0.0 | 3.4 | 24.5 | 24.4 | 13.3 | 4.0 | - | ND |
| | | 15 | 1.7 | 0.6 | 13.1 | 9.0 | 21.3 | 13.3 | 3.7 | - | ND |
| APTS | 2 | 5 | 0.0 | 0.0 | 11.4 | 21.1 | 24.8 | 7.5 | 1.1 | - | 0.33 |
| APTS | 5 | 2 | 6.5 | 0.1 | 2.1 | 25.4 | 28.3 | 12.8 | 1.9 | - | ND |
| | | 10 | 3.2 | 0.2 | 8.7 | 12.9 | 27.0 | 11.8 | 1.8 | - | ND |
| CuCl | 1 | 2 | 4.7 | 0.0 | 4.7 | 19.5 | 23.4 | 16.7 | 5.0 | - | ND |
| | | 10 | 1.7 | 0.3 | 9.2 | 10.6 | 20.0 | 17.7 | 5.3 | - | ND |
| CuCl | 5 | 5 | 4.2 | 0.0 | 3.7 | 19.8 | 21.6 | 12.8 | 5.1 | - | ND |
| | | 20 | 1.6 | 0.3 | 10.6 | 9.0 | 20.1 | 12.6 | 5.6 | - | ND |

### Exemple 6 : Influence de l'addition d'un solvant sur la réaction de la cyclisation de l'ester dimérisé.

Afin d'augmenter le rendement de production de lactide, nous avons ajouté un solvant inerte de manière à diluer le milieu réactionnel et à augmenter le libre parcours moyen. Dans cet objectif, un solvant susceptible de recristalliser le lactide à froid a été choisi. Il s'agit du p-cymène (isopropyltoluène) dont la température d'ébullition permet, par ailleurs, de balayer une large gamme de température (Téb=178°C). Le tableau 6 résume les résultats pour différentes dilutions lorsque la réaction de cyclisation est catalysée par Sn(Oct)2 à 145°C dans un réacteur batch.
Comme attendu, la dilution du milieu de cyclisation favorise la formation de lactide et limite fortement les réactions intermoléculaires de transestérification, d'ouverture de cycle et même de racémisation. Néanmoins le rendement en lactide reste faible et la présence de polymére omniprésente. Dés lors, si le principe même d'augmenter le libre parcours moyen des molécules via l'addition d'un solvant tient la route, il est préférable de se tourner vers d'autres catalyseurs plus sélectifs, tels que l'APTS ou encore le chlorure de cuivre (I).

**Tableau 6:**

| *Influence de l'addition de p-cymène sur la réaction de formation de lactide au départ de lactyllactate d'éthyle (LEt 5.4, L2Et 77.0, L3Et 16.2, L4Et 1.0, L5Et 0.3) dans un réacteur de type batch à sous flux d'azote et en présence de 1 %pds Sn(Oct)2*. | | | | | | | | | | |
|---|---|---|---|---|---|---|---|---|---|---|
| M solv (%pds) | t (min) | Gc normalisé en fonction des distillats | | | | | | | PLA | Acid. (mmol /g) |
| | | LEt | m-LD | L.L-LD | L2Et | L3Et | L4Et | L5Et | | |
| 0^{b} | 15 | 8.9 | 1.1 | 5.2 | 28.5 | 28.2 | 14.8 | 1.1 | + | 0.17 |
| | 25 | 6.8 | 1.0 | 5.4 | 23.1 | 25.7 | 18.4 | 3.5 | + | ND |
| | 30 | 1.9 | 1.2 | 4.3 | 18.8 | 27.1 | 19.4 | 5.6 | + | ND |
| 80^{a} | 5 | 0.0 | 0.0 | 5.5 | 64.8 | 22.7 | 0.0 | 0.0 | + | ND |
| 95^{b} | 5 | 0.0 | 0.0 | 7.6 | 81.8 | 4.1 | 1.5 | 0.5 | + | ND |
| | 10 | 0.0 | 0.0 | 10.2 | 74.5 | 7.6 | 2.1 | 1.0 | + | ND |
| | 15 | 0.0 | 0.0 | 12.3 | 74.8 | 9.0 | 2.3 | 1.6 | + | <0.01 |

| | | | | | | | | | | |
|---|---|---|---|---|---|---|---|---|---|---|
| ^{a}-Après 12 min, le milieu est trop visqueux pour permettre une analyse GC. | | | | | | | | | | |
| ^{b}-Au delà du dernier temps de prélèvement, le milieu devient trop visqueux pour effectuer une analyse GC. Msolv : masse de solvant | | | | | | | | | | |

### Exemple 7 : Cyclisation sélective en milieu dilué et purification du lactide obtenu.

L'exemple précédent ayant démontré le bien fondé du principe de dilution du milieu réactionnel par un solvant inerte, nous avons effectué la même expérience en ayant prix soin toutefois de remplacer le principe actif afin de confirmer l'aspect sélectif de la réaction. En effet, les résultats du tableau 7 montre qu'un choix judicieux du catalyseur, à savoir un catalyseur qui ne favorise pas les réactions de transestérification intermoléculaire et la polymérisation du lactide, permet de maintenir une sélectivité et une stéréospécificité au procédé. Dans la mesure où la composition en lactate d'éthyle diminue de maniére constante au fur et à mesure de la formation de lactide, il apparaît clair que le mécanisme mis en jeu est bien la cyclisation du dimère linéaire par opposition a un mécanisme de dépolymérisation.

La température du mélange obtenu après 30 min de réaction avec l'APTS comme catalyseur, a été ramenée à la température ambiante, ce qui a eu pour effet d'insolubiliser le lactide qui a pu être extrait par simple filtration (72% du lactide ont pu être récupérés). Le lactide récolté après filtration s'est avéré avoir une pureté déterminée par GC de 96.3% en L-LD (il reste 2.5% de p-cymène) et une acidité déterminée par titrage en milieu non aqueux de 20 méq/kg.

**Tableau 7:**

| *Composition des produits de dimérisation du lactyllactate d'éthyle ( L2Et 76.8%) en lactide dans un réacteur de type batch sous flux d'azote et en présence de 95 % en poids de p-cymène et 1 % en poids de catalyseur.* | | | | | | | | | | | |
|---|---|---|---|---|---|---|---|---|---|---|---|
| Calalyseur | M catalyseur (% pds) | t (min) | GC normalisé en fonction des distillats | | | | | | | (%) PLA | Acide (mmol /g) |
| | | | LEt | m-LD | L.L-LD | L2Et | L3Et | L4Et | L5Et | | |
| APTS | 1 | 10 | 0.3 | 0.7 | 18.3 | 76.95 | 2.5 | 1.2 | 0.0 | - | ND |
| | | 20 | 0.3 | 0.4 | 45.8 | 52.7 | 0.0 | 0.7 | 0.0 | - | ND |
| | | 30 | 0.4 | 1.7 | 44.5 | 47.2 | 5.2 | 0.9 | 0.0 | - | ND |
| CuCl | 5 | 10 | 0.2 | 1.6 | 12.6 | 84.7 | 0.0 | 0.9 | 0.0 | - | ND |
| | | 15 | 0.2 | 1.7 | 15.1 | 80.7 | 0.9 | 1.4 | 0.0 | - | ND |
| | | 20 | 0.1 | 2.0 | 27.5 | 68.4 | 0.5 | 1.4 | 0.0 | - | ND |
| | | 30 | 0.3 | 2.1 | 42.1 | 53.6 | 0.6 | 1.2 | 0.0 | - | ND |

## Revendications

1. Procédé de production d'esters cycliques dimériques au départ d'un brut d'estérification partielle, comprenant les étapes suivantes :
(a) obtention d'un brut d'estérification partielle, à savoir un mélange d'acide lactique et/ou glycolique et d'un ester lactique et/ou glycolique;
(b) dimérisation de la source initiale issue de (a) en un dimère linéaire de l'ester par réaction de condensation entre la fonction hydroxyle de l'ester et la fonction acide carboxylique de l'acide;
(c) cyclisation des dimères linéaires générés en (b) en esters cycliques dimériques en favorisant les réactions de transestérification intramoléculaire au détriment des réactions intermoléculaires;
(d) extraction de l'ester cyclique dimérique brut généré en (c) et récupération de l'ester cyclique dimérique purifié.

2. Procédé suivant la revendication 1, **caractérisé en ce que** le brut d'estérification partielle est obtenu par mélange des produits purs ou, avantageusement, extrait à un moment bien déterminé de l'étape d'estérification de l'acide lactique et/ou glycolique en ester.

3. Procédé suivant l'une quelconque des revendications 1 et 2, **caractérisé en ce que** le brut d'estérification partielle correspond à une composition relative en ester et en acide sous forme monomérique comprise entre 50 et 99% en ester et préférablement entre 70 et 95%.

4. Procédé suivant l'une quelconque des revendications précédentes, **caractérisé en ce que** l'étape de dimérisation (b) est réalisée à des températures n'excédant pas 190°C, préférablement 175°C, plus préférablement inférieures à 165°C et plus préférablement encore inférieures à 150°C.

5. Procédé suivant l'une quelconque des revendications précédentes, **caractérisé en ce que** l'étape de dimérisation (b) comporte une réactivité totale vis-à-vis de l'acide lactide et/ou glycolique, acidité résiduelle due à l'acide nulle, et une teneur en oligomère de l'ester lactique et/ou glycolique dans les produits de réaction inférieure à 10%, préférablement inférieure à 5% et plus préférablement encore inférieure à 2%.

6. Procédé suivant la revendication 5, dans lequel les oligomères de l'ester se présentent exclusivement sous la forme de trimère ou de tétramère de l'ester lactique.

7. Procédé suivant l'une quelconque des revendications précédentes, **caractérisé en ce que** l'étape de dimérisation (b) est initiée par un catalyseur d'estérification, choisi parmi les catalyseurs acides, solides et hétérogene; les catalyseurs à base de sel organique de métal; les catalyseurs acides et solubles; les catalyseurs micéllaires, les catalyseurs acides échangeurs d'ions ; les phosphates ; les catalyseurs à base de silice; les enzymes; les zéolites ; les catalyseurs non polaires et enfin les sels.

8. Procédé suivant la revendication 7, **caractérisé en ce que** le catalyseur est un acide de Brönsted nécessitant une faible énergie d'activation et favorisant les réactions d'estérification plutôt que de transestérification comme par exemple, l'acide para-toluène sulfonique (APTS).

9. Procédé suivant l'une quelconque des revendications 7 ou 8, **caractérisé en ce que** les teneurs en catalyseur sont comprises entre 0 et 5% et préférablement comprises entre 0.1 et 2%.

10. Procédé suivant l'une quelconque des revendications précédentes, **caractérisé en ce que** l'étape de dimérisation (b) comprend l'utilisation d'un réacteur offrant une capacité de volatilisation importante et un temps de séjour compris entre 0.1 et 60 min, préférablement compris entre 0.1 et 30 min, plus préférablement compris entre 0 1 et 15 min et plus préférablement encore compris entre 0.1 et 5 min tel que par exemple un réacteur en couche mince.

11. Composition de dimères linéaires d'ester lactique et/ou glycolique obtenue à l'étape (b) **caractérisée en ce qu'**elle comporte entre 50 et 100% d'ester linéaire dimérisé et préférablement entre 70 et 100%, et une teneur en oligomère de l'ester lactique inférieure à 15%, préférablement inférieure à 10%, plus préférablement inférieure à 5% et plus préférablement encore inférieure à 2%.

12. Procédé suivant l'une quelconque des revendications 1 à 10, **caractérisé en ce que** l'étape (c) consiste exclusivement en un processus de cyclisation du dimère linéaire et non en un processus de dépolymérisation d'oligomères d'ester lactique et/ou glycolique.

13. Procédé suivant l'une quelconque des revendications de procédés précédentes, **caractérisé en ce que** l'étape de cyclisation (c) est réalisée à des températures n'excédant pas 190°C, préférablement inférieures à 175°C et plus préférablement encore inférieures à 165°C.

14. Procédé suivant l'une quelconque des revendications précédentes, **caractérisé en ce que** dans l'étape de cyclisation (c) on favorise les réactions intramoléculaires des molécules de dimères linéaires et on diminue la probabilité de réactions intermoléculaires.

15. Procédé suivant la revendication 14, **caractérisé en ce que** pour favoriser les réactions intramoléculaires des molécules de dimères linéaires, on réalise la réaction soit dans un solvant de préférence inerte, soit en phase vapeur avec ou sans gaz vecteur.

16. Procédé suivant la revendication 15, **caractérisé en ce que** le solvant est choisi parmi les cétones, les éthers, les solvants aromatiques, aliphatiques, les solvants à base de silicone ou les solvants halogénés.

17. Procédé suivant l'une quelconque des revendications 15 ou 16, **caractérisé en ce que** le solvant est susceptible de recristalliser l'ester cyclique dimérique à froid.

18. Procédé suivant l'une quelconque des revendications précédentes, **caractérisé en ce que** l'étape de cyclisation (c) est initiée par un catalyseur d'estérification, choisi parmi les catalyseurs acides, solides et hétérogènes; les catalyseurs à base de sel organique de métal; les catalyseurs acides et solubles; les catalyseurs micéllaires, les catalyseurs acides échangeurs d'ions ; les phosphates ; les catalyseurs à base de silice; les enzymes; les zéolites ; les catalyseurs non polaires et enfin les sels.

19. Procédé suivant la revendication 18, **caractérisé en ce que** le catalyseur est l'acide para-toluène sulfonique (APTS) ou le chlorure de cuivre (CuCl).

20. Procédé suivant l'une quelconque des revendications 18 ou 19, **caractérisé en ce que** les teneurs en catalyseur sont comprises entre 0 et 5% et préférablement comprises entre 0.1 et 2%.

21. Procédé suivant l'une quelconque des revendications de procédé précédentes, **caractérisé en ce que** l'étape de cyclisation (c) comprend l'utilisation d'un réacteur offrant une capacité d'extraction importante d'un composé volatile et un temps de séjour compris entre 0.1 et 120 min, préférablement compris entre 0.1 et 60 min, et plus préférablement encore compris entre 0.1 et 30 min.

22. Procédé suivant l'une quelconque des revendications de procédé précédentes. **caractérisé en ce que** l'étape (d) comporte une double distillation fractionnée de l'ester cyclique dimérique brut obtenu en (c) permettant d'obtenir un ester cyclique dimérique d'une très grande pureté chimique.

23. Procédé suivant la revendication 22, **caractérisé en ce que** la seconde colonne de distillation et/ou la première colonne de distillation comporte un procédé flash.

24. Procédé suivant l'une quelconque des revendications de procédé precédentes. **caractérisé en ce que** l'étape (d) comporte une distillation fractionnée, avec ou sans procédé flash incorporé ou non dans le ballon de distillation, couplée à une recristallisation en milieu fondu de l'ester cyclique dimérique brut obtenu en (c).

25. Procédé suivant l'une quelconque des revendications de procédé précédentes, **caractérisé en ce que** l'étape (d) comporte une recristallisation de l'ester cyclique dimérique brut obtenu en (c) dans un solvant.

26. Procédé suivant la revendication 25, **caractérise en ce que** le solvant de recristallisation est le solvant inerte introduit lors de l'étape de cyclisation (c) ou un second solvant issu de la même famille et choisi parmi les cétones, les éthers, les composés aromatiques et aliphatiques, les composés à base de silicone et les composés halogénés.

27. Procédé suivant l'une quelconque des revendications précédentes, **caractérisé en ce que** l'ester cyclique dimérique est le lactide.

28. Composition d'esters cycliques dimériques obtenue à l'étape (c) d'un procédé selon la revendication 27, **caractérisée en ce qu'**elle comporte entre 0 et 80% de monomère et dimère linéaire, préférablement entre 10 et 60%, et plus préférablement encore comprise entre 10 et 40%, une teneur en méso-LD comprise entre 0 et 5%, préférablement comprise entre 0 et 2% et plus préférablement encore inférieure à 1%, et une teneur en trimère et tétramère inférieure à 15%, préférablement inférieure à 10%, plus préférablement inférieure à 5% et plus préférablement encore inférieure à 2%, le solde étant constitué de L-LD.

29. Procédé suivant la revendication 27, **caractérisé en ce que** la pureté en L-lactide est comprise entre 99.0 et 99.9, préférablement comprise entre 99.5 et 99.8, la purete en méso-lactide étant comprise entre 0 et 2%, préférablement entre 0 et 0.5%, plus préférablement comprise entre 0 et 0.2%, et plus préférablement encore entre 0 et 0.1%.

## Patentansprüche

1. Verfahren zur Herstellung von dimeren zyklischen Estern ausgehend von einem Rohprodukt einer partiellen Veresterung, umfassend die folgenden Schritte:
(a) Gewinnung eines Rohprodukts einer partiellen Veresterung, nämlich einer Mischung aus Milchsäure und/oder Glykolsäure und einem Milchsäure- und/oder Glykolsäureester;
(b) Dimerisierung des in (a) erhaltenen Ausgangsmaterials zu einem geradkettigen Dimeren des Esters durch Kondensation zwischen der Hydroxyfunktion des Esters und der Carbonsäurefunktion der Säure;
(c) Zyklisierung der in (b) hergestellten geradkettigen Dimeren zu dimeren zyklischen Estern unter Begünstigung der intramolekularen Umesterungsreaktionen gegenüber den intermolekularen Reaktionen;
(d) Extraktion des in (c) hergestellten rohen dimeren zyklischen Esters und Gewinnung des gereinigten dimeren zyklischen Esters.

2. Verfahren nach Anspruch 1, **dadurch gekennzeichnet, daß** das Rohprodukt der partiellen Veresterung durch Mischen von reinen Produkten oder vorteilhaft durch Entfernen zu einem genau bestimmten Zeitpunkt des Schritts der Veresterung der Milchsäure und/oder Glykolsäure zum Ester erhalten wird.

3. Verfahren nach Anspruch 1 oder 2, **dadurch gekennzeichnet, daß** das Rohprodukt der partiellen Veresterung eine relative Zusammensetzung aus Ester und Säure in monomerer Form von 50 bis 99% Ester, vorzugsweise 70 bis 95% Ester, aufweist.

4. Verfahren nach einem der vorhergehenden Ansprüche, **dadurch gekennzeichnet, daß** der Dimerisationsschritt (b) bei Temperaturen, die 190°C nicht überschreiten, vorzugsweise 175°C, stärker bevorzugt unter 165°C und noch stärker bevorzugt unter 150°C, durchgeführt wird.

5. Verfahren nach einem der vorhergehenden Ansprüche, **dadurch gekennzeichnet, daß** bei dem Dimerisationsschritt (b) ein vollständiger Umsatz der Milchsäure und/oder Glykolsäure erzielt wird, die restliche Acidität aufgrund der Säure Null beträgt und der Gehalt an Oligomeren des Milchsäureund/oder Glykolsäureesters in den Reaktionsprodukten unter 10%, vorzugsweise unter 5%, noch stärker bevorzugt unter 2%, liegt.

6. Verfahren nach Anspruch 5, wobei die Oligomere des Esters ausschließlich in Form von Trimeren oder Tetrameren des Milchsäure- und/oder Glykolsäureesters vorliegen.

7. Verfahren nach einem der vorhergehenden Ansprüche, **dadurch gekennzeichnet, daß** der Dimerisationsschritt (b) durch einen Veresterungskatalysator aus der Gruppe saure, feste und heterogene Katalysatoren, Katalysatoren auf Basis von organischen Metallsalzen, sauren und löslichen Katalysatoren, miellenartigen Katalysatoren, sauren Ionenaustauscherkatalysatoren, Phosphate, Katalysatoren auf Silikabasis, Enzyme, Zeolithe, unpolare Katalysatoren sowie schließlich Salze eingeleitet wird.

8. Verfahren nach Anspruch 7, **dadurch gekennzeichnet, daß** es sich bei dem Katalysator um eine Brönsted-Säure handelt, die nur wenig Aktivierungsenergie benötigt und Veresterungsreaktionen gegenüber Umesterungsreaktionen begünstigt, wie zum Beispiel para-Toluolsulfonsäure (PTSA).

9. Verfahren nach Anspruch 7 oder 8, **dadurch gekennzeichnet, daß** die Katalysatorgehalte zwischen 0 und 5%, vorzugsweise zwischen 0,1 und 2%, liegen.

10. Verfahren nach einem der vorhergehenden Ansprüche, **dadurch gekennzeichnet, daß** bei dem Dimerisationsschritt (b) ein Reaktor mit einer hohen Verflüchtigungskapazität und einer Verweildauer von 0,1 bis 60 Minuten, vorzugsweise 0,1 bis 30 Minuten, stärker bevorzugt 0,1 bis 15 Minuten, noch stärker bevorzugt 0,1 bis 5 Minuten, wie zum Beispiel ein Dünnschichtreaktor, verwendet wird.

11. Zusammensetzung von geradkettigen Dimeren von Milchsäure- und/oder Glykolsäureester, die in Schritt (b) erhalten wird, **dadurch gekennzeichnet, daß** sie 50 bis 100%, vorzugsweise 70 bis 100%, dimerisierten geradkettigen Ester sowie einen Gehalt an Oligomeren von Milchsäure- und/oder Glykolsäureester unter 15%, vorzugsweise unter 10%, stärker bevorzugt unter 5%, noch stärker bevorzugt unter 2%, aufweist.

12. Verfahren nach einem der Ansprüche 1 bis 10, **dadurch gekennzeichnet, daß** Schritt (c) ausschließlich aus einer Zyklisierung des geradkettigen Dimeren und nicht aus einer Entpolymerisation von Oligomeren des Milchsäureund/oder Glykolsäureesters besteht.

13. Verfahren nach einem der vorhergehenden Verfahrensansprüche, **dadurch gekennzeichnet, daß** der Zyklisierungsschritt (c) bei Temperaturen, die 190°C nicht überschreiten, vorzugsweise unter 175°C, noch stärker bevorzugt unter 165°C, durchgeführt wird.

14. Verfahren nach einem der vorhergehenden Ansprüche, **dadurch gekennzeichnet, daß** man im Zyklisierungsschritt (c) die intramolekularen Reaktionen der Moleküle der geradkettigen Dimeren gegenüber der Wahrscheinlichkeit von intermolekularen Reaktionen begünstigt.

15. Verfahren nach Anspruch 14, **dadurch gekennzeichnet, daß** man zur Begünstigung der intramolekularen Reaktionen der Moleküle von geradkettigen Dimeren die Reaktion entweder in einem Lösungsmittel, vorzugsweise einem inerten Lösungsmittel, oder in der Dampfphase mit oder ohne Trägergas durchführt.

16. Verfahren nach Anspruch 15, **dadurch gekennzeichnet, daß** das Lösungsmittel aus der Gruppe Ketone, Ether, aromatische oder aliphatische Lösungsmittel, Lösungsmittel auf Silikonbasis oder halogenierte Lösungsmittel stammt.

17. Verfahren nach Anspruch 15 oder 16, **dadurch gekennzeichnet, daß** mit dem Lösungsmittel der dimere zyklische Ester in der Kälte umkristallisiert werden kann.

18. Verfahren nach einem der vorhergehenden Ansprüche, **dadurch gekennzeichnet, daß** der Zyklisierungsschritt (c) durch einen Veresterungskatalysator aus der Gruppe saure, feste und heterogene Katalysatoren, Katalysatoren auf Basis von organischen Metallsalzen, sauren und löslichen Katalysatoren, miellenartigen Katalysatoren, sauren Ionenaustauscherkatalysatoren, Phosphate, Katalysatoren auf Silikabasis, Enzyme, Zeolithe, unpolare Katalysatoren sowie schließlich Salze eingeleitet wird.

19. Verfahren nach Anspruch 18, **dadurch gekennzeichnet, daß** es sich bei dem Katalysator um para-Toluolsulfonsäure (PTSA) oder Kupferchlorid (CuCl) handelt.

20. Verfahren nach Anspruch 18 oder 19, **dadurch gekennzeichnet, daß** die Katalysatorgehalte zwischen 0 und 5%, vorzugsweise zwischen 0,1 und 2%, liegen.

21. Verfahren nach einem der vorhergehenden Verfahrensansprüche, **dadurch gekennzeichnet, daß** im Zyklisierungsschritt (c) ein Reaktor mit einer hohen Kapazität zur Entfernung einer flüchtigen Verbindung und einer Verweildauer von 0,1 bis 120 Minuten, vorzugsweise 0,1 bis 60 Minuten, noch stärker bevorzugt 0,1 bis 30 Minuten, verwendet wird.

22. Verfahren nach einem der vorhergehenden Verfahrensansprüche, **dadurch gekennzeichnet, daß** Schritt (d) eine zweifache fraktionierte Destillation des in (c) erhaltenen rohen dimeren zyklischen Esters umfaßt, wodurch man einen dimeren zyklischen Ester in sehr hoher chemischer Reinheit erhält.

23. Verfahren nach Anspruch 22, **dadurch gekennzeichnet, daß** die zweite Destillationskolonne und/oder die erste Destillationskolonne ein Flash-Verfahren beinhaltet.

24. Verfahren nach einem der vorhergehenden Verfahrensansprüche, **dadurch gekennzeichnet, daß** Schritt (d) eine fraktionierte Destillation mit oder ohne in den Destillationskolben integriertes oder nicht integriertes Flash-Verfahren, die mit einer Umkristallisation in der Schmelze des in (c) erhaltenen rohen dimeren zyklischen Esters gekoppelt ist, umfaßt.

25. Verfahren nach einem der vorhergehenden Verfahrensansprüche, **dadurch gekennzeichnet, daß** Schritt (d) eine Umkristallisation des in (c) erhaltenen rohen dimeren zyklischen Esters in einem Lösungsmittel beinhaltet.

26. Verfahren nach Anspruch 25, **dadurch gekennzeichnet, daß** es sich bei dem Lösungsmittel für die Umkristallisation um das bei dem Zyklisierungsschritt (c) eingeführte inerte Lösungsmittel oder um ein zweites Lösungsmittel aus der gleichen Familie aus der Gruppe der Ketone, Ether, aromatischen und aliphatischen Verbindungen, Verbindungen auf Silikonbasis und halogenierten Verbindungen handelt.

27. Verfahren nach einem der vorhergehenden Ansprüche, **dadurch gekennzeichnet, daß** es sich bei dem dimeren zyklischen Ester um das Lactid handelt.

28. Zusammensetzung aus dimeren zyklischen Estern, die in Schritt (c) eines Verfahrens nach Anspruch 27 erhalten wurden, **dadurch gekennzeichnet, daß** sie 0 bis 80%, vorzugsweise 10 bis 60%, noch stärker bevorzugt 10 bis 40%, geradkettige Dimere und Monomere enthält, einen meso-LD-Gehalt von 0 bis 5%, vorzugsweise 0 bis 2%, noch stärker bevorzugt unter 1%, und einen Trimeren- und Tetramerengehalt unter 15%, vorzugsweise unter 10%, stärker bevorzugt unter 5%, noch stärker bevorzugt unter 2%, aufweist, wobei der Rest aus L-LD besteht.

29. Verfahren nach Anspruch 27, **dadurch gekennzeichnet, daß** die L-Lactid-Reinheit 99,0 bis 99,9, vorzugsweise 99,5 bis 99,8, beträgt und das meso-Lactid-Gehalt 0 bis 2%, vorzugsweise 0 bis 0,5%, stärker bevorzugt 0 bis 0,2%, noch stärker bevorzugt 0 bis 0,1%, beträgt.

## Claims

1. Process for the production of dimeric cyclic esters starting from a crude partial esterification product, comprising the following stages:
(a) production of a crude partial esterification product, namely a mixture of lactic and/or glycolic acid and of a lactic and/or glycolic ester;
(b) dimerization of the initial source resulting from (a) to give a linear dimer of the ester by a condensation reaction between the hydroxyl functional group of the ester and the carboxylic acid functional group of the acid;
(c) cyclization of the linear dimers generated in (b) to give dimeric cyclic esters by promoting intramolecular transesterification reactions at the expense of intermolecular reactions;
(d) extraction of the crude dimeric cyclic ester generated in (c) and recovery of the purified dimeric cyclic ester.

2. Process according to Claim 1, **characterized in that** the crude partial esterification product is obtained by mixing the pure products or, advantageously, extracted at a very specific moment in the stage of esterification of lactic and/or glycolic acid to give the ester.

3. Process according to either one of Claims 1 and 2, **characterized in that** the crude partial esterification product corresponds to a relative composition of ester and of acid in the monomeric form of between 50 and 99% of ester and preferably between 70 and 95%.

4. Process according to any one of the preceding claims, **characterized in that** the dimerization stage (b) is carried out at temperatures not exceeding 190°C, preferably 175°C, more preferably of less than 165°C and more preferably still of less than 150°C.

5. Process according to any one of the preceding claims, **characterized in that** the dimerization stage (b) comprises complete reaction of the lactic and/or glycolic acid, no residual acidity due to the acid and a content of oligomer of the lactic and/or glycolic ester in the reaction products of less than 10%, preferably of less than 5% and more preferably still of less than 2%.

6. Process according to Claim 5, in which the oligomers of the ester exist solely in the form of trimer or of tetramer of the lactic and/or glycolic ester.

7. Process according to any one of the preceding claims, **characterized in that** the dimerization stage (b) is initiated with an esterification catalyst chosen from solid and heterogeneous acid catalysts; catalysts based on organic metal salt; soluble acid catalysts; micellar catalysts; ion-exchange acid catalysts; phosphates; silica-based catalysts; enzymes; zeolites; nonpolar catalysts and, finally, salts.

8. Process according to Claim 7, **characterized in that** the catalyst is a Brönsted acid requiring a low energy of activation and promoting esterification reactions rather than transesterification reactions, such as, for example, para-toluenesulphonic acid (PTSA).

9. Process according to either one of Claims 7 and 8, **characterized in that** the contents of catalyst are between 0 and 5% and preferably between 0.1 and 2%.

10. Process according to any one of the preceding claims, **characterized in that** the dimerization stage (b) comprises the use of a reactor offering a large volatilization capacity and a residence time of between 0.1 and 60 min, preferably of between 0.1 and 30 min, more preferably of between 0.1 and 15 min and more preferably still of between 0.1 and 5 min, such as, for example, a thin film reactor.

11. Composition comprising linear dimers of lactic and/or glycolic ester obtained in stage (b), **characterized in that** it comprises between 50 and 100% of dimerized linear ester and preferably between 70 and 100% and a content of oligomer of the lactic and/or glycolic ester of less than 15%, preferably of less than 10%, more preferably of less than 5% and more preferably still of less than 2%.

12. Process according to any one of Claims 1 to 10, **characterized in that** stage (c) consists solely of a process for the cyclization of the linear dimer and not of a process for the depolymerization of oligomers of lactic and/or glycolic ester.

13. Process according to any one of the preceding process claims, **characterized in that** the cyclization stage (c) is carried out at temperatures not exceeding 190°C, preferably of less than 175°C and more preferably still of less than 165°C.

14. Process according to any one of the preceding claims, **characterized in that**, in the cyclization stage (c), the intramolecular reactions of the molecules of linear dimers are promoted and the probability of intermolecular reactions is reduced.

15. Process according to Claim 14, **characterized in that**, in order to promote the intramolecular reactions of the molecules of linear dimers, the reaction is carried out either in a solvent, preferably an inert solvent, or in the vapour phase, with or without a carrier gas.

16. Process according to Claim 15, **characterized in that** the solvent is chosen from ketones, ethers, aromatic or aliphatic solvents, silicone-based solvents or halogenated solvents.

17. Process according to either one of Claims 15 and 16, **characterized in that** the solvent is capable of recrystallizing the dimeric cyclic ester under cold conditions.

18. Process according to any one of the preceding claims, **characterized in that** the cyclization stage (c) is initiated with an esterification catalyst chosen from solid and heterogeneous acid catalysts; catalysts based on organic metal salt; soluble acid catalysts; micellar catalysts; ion-exchange acid catalysts; phosphates; silica-based catalysts; enzymes; zeolites; nonpolar catalysts and, finally, salts.

19. Process according to Claim 18, **characterized in that** the catalyst is para-toluenesulphonic acid (PTSA) or copper chloride (CuCl).

20. Process according to either one of Claims 18 and 19, **characterized in that** the contents of catalysts are between 0 and 5% and preferably between 0.1 and 2%.

21. Process according to any one of the preceding process claims, **characterized in that** the cyclization stage (c) comprises the use of a reactor offering a large extraction capacity for a volatile compound and a residence time of between 0.1 and 120 min, preferably of between 0.1 and 60 min and more preferably still of between 0.1 and 30 min.

22. Process according to any one of the preceding process claims, **characterized in that** stage (d) comprises a double fractional distillation of the crude dimeric cyclic ester obtained in (c), making it possible to obtain a dimeric cyclic ester of very high chemical purity.

23. Process according to Claim 22, **characterized in that** the second distillation column and/or the first distillation column comprises a flash process.

24. Process according to any one of the preceding process claims, **characterized in that** stage (d) comprises a fractional distillation, with or without a flash process incorporated or not incorporated in the distillation flask, coupled to a recrystallization in a molten medium, of the crude dimeric cyclic ester obtained in (c).

25. Process according to any one of the preceding process claims, **characterized in that** stage (d) comprises a recrystallization of the crude dimeric cyclic ester obtained in (c) from a solvent.

26. Process according to Claim 25, **characterized in that** the recrystallization solvent is the inert solvent introduced during the cyclization stage (c) or a second solvent from the same family and chosen from ketones, ethers, aromatic and aliphatic compounds, silicone-based compounds and halogenated compounds.

27. Process according to any one of the preceding claims, **characterized in that** the dimeric cyclic ester is lactide.

28. Composition comprising dimeric cyclic esters which is obtained in stage (c) of a process according to Claim 27, **characterized in that** it comprises between 0 and 80% of monomer and linear dimer, preferably between 10 and 60% and more preferably still between 10 and 40%, a content of meso-LD of between 0 and 5%, preferably between 0 and 2% and more preferably still of less than 1%, and a content of trimer and tetramer of less than 15%, preferably of less than 10%, more preferably of less than 5% and more preferably still of less than 2%, the balance being composed of L-LD.

29. Process according to Claim 27, **characterized in that** the purity of L-lactide is between 99.0 and 99.9, preferably between 99.5 and 99.8, the content of meso-lactide being between 0 and 2%, preferably between 0 and 0.5%, more preferably between 0 and 0.2% and more preferably still between 0 and 0.1%.
